Europäisches Patentamt

European Patent Office

Office européen des brevets

(11) Publication number: **0 380 102**
**A1**

(12) **EUROPEAN PATENT APPLICATION**

(21) Application number: **90101510.7**

(22) Date of filing: **25.01.90**

(51) Int. Cl.5: **A61M 29/02, A61M 25/10,**
**A61L 29/00**

(30) Priority: **26.01.89 US 302584**

(43) Date of publication of application:
**01.08.90 Bulletin 90/31**

(84) Designated Contracting States:
**CH DE FR GB IT LI NL**

(71) Applicant: **ADVANCED CARDIOVASCULAR SYSTEMS, INC.**
**3200 Lakeside Drive**
**Santa Clara California 95052(US)**

(72) Inventor: **Hattori, Sachiko**
**998 Inverness Way**
**Sunnyval California 94087(US)**
Inventor: **Kamdar, Kirti**
**370 W. Olive Street**
**Sunnyvale California 94086(US)**
Inventor: **Hart, Jerry A.**
**102 Lakeridge Circle**
**Fallbrook California 92028(US)**

(74) Representative: **Baillie, Iain Cameron et al**
**c/o Ladas & Parry Isartorplatz 5**
**D-8000 München 2(DE)**

(54) **Vascular catheter with durable lubricious coating.**

(57) A vascular catheter (10), particularly a dilatation catheter for angioplasty procedures, having bonded onto the surface thereof a durable lubricious coating (17). The coating (17) preferably has two layers (26, 28), a first polymer layer (26) such as polysiloxane comprising a three-dimensional network of polymer units, which is bonded to the catheter surface, and a second lubricious layer (28) such as silicone oil which is bonded to the first layer (26). Substantial reductions in frictional coefficients and effective profiles are obtained.

FIG. 1

FIG. 2

## VASCULAR CATHETER WITH DURABLE LUBRICIOUS COATING

## BACKGROUND OF THE INVENTION

This invention generally relates to a vascular catheter having a durable lubricious coating on the surface thereof and particularly to such catheters suitable for percutaneous transluminal coronary angioplasty (PTCA).

In classical PTCA procedures, a guiding catheter having a preformed distal tip is percutaneously introduced into the cardiovascular system of a patient and advanced therein until the distal tip of the guiding catheter is in the ostium of the desired coronary artery. A guidewire and a balloon dilatation catheter are introduced into the patient's vascular system through the previously introduced guiding catheter. The guidewire is first advanced out of the distal tip of the guiding catheter into the patient's coronary vasculature until the distal end of the guidewire crosses the lesion to be dilated. The dilatation catheter, having an inflatable balloon on the distal portion thereof, is advanced over the previously positioned guidewire, with the guidewire slidably disposed within an inner lumen of the dilatation catheter, until the balloon thereof is properly positioned across the lesion. Once in position across the lesion, the flexible, relatively inelastic balloon is inflated to a predetermined size with radiopaque liquid at relatively high pressures (e.g., at least 100 psi) to radially compress the atherosclerotic plaque of the lesion against the inside of the artery wall. The balloon is then deflated so that the blood flow can resume through the dilated artery and the catheter can be removed.

Further details of the aforesaid angioplasty procedures and the devices used in such procedures, can be found in U.S. Patent 4,332,254 (Lundquist); U.S. Patent 4,323,071 (Simpson-Robert); U.S. Patent 4,468,224; (Enzmann et al.) and U.S. Patent 4,616,652 (Simpson) which are hereby incorporated herein in their entirety.

Steerable dilatation catheters with built-in guidewires or guiding elements are being used with greater frequency because they have better pushability, they are easily steerable and their deflated profiles are smaller than conventional dilatation catheters having the same inflated balloon size. The details of low-profile steerable dilatation catheters may be found in U. S. Patent 4,582,181 (Samson), U. S. Patent 4,664,113 (Frisbie), and U. S. Patent 4,641,654 (Samson et al.) and pending U. S. applications Serial Nos. 000,650 filed January 6, 1987, and 000,648 filed January 6, 1987, which are hereby incorporated in their entirety by reference thereto.

It has generally been the trend to make the deflated profiles of dilatation catheters as small as possible because a smaller profile allows a catheter to pass through tighter lesions and to be advanced much further into the patient's coronary anatomy. Various steps have been taken to reduce the profile of dilatation catheters but there are limits to what can be done. For example, as the diameter of the guidewire is reduced in order to reduce the profile of the catheter, the ability of the guidewire to transmit torsional and axial forces is likewise reduced. Moreover, as the diameter of a guidewire is reduced, the tendency for buckling when being advanced through tortuous coronary vasculature is increased.

Reducing the wall thickness of the tubular members which make up the catheters, particularly the balloon wall, can also reduce the profile, but as with reducing the diameter of the guidewire, there is a limit on how much the wall thickness of the tubular members can be reduced without detrimentally affecting the performance of the catheter. Reducing the thickness of the balloon wall below certain limits can greatly increase the occurrence of undesirable pinholes, even though the basic strength of the balloon material may be sufficient to prevent rupture.

Efforts have been made to reduce the surface friction of vascular catheters such as by applying lubricating oils or forming a hydrogel coating thereon. However, these coatings have not been found to be very durable. When a catheter having such a lubricious coating passes through tortuous arterial passageways, such as those found in the coronary anatomy, the lubricious properties are greatly reduced or lost.

What has been needed and heretofore unavailable is a vascular catheter, particularly a dilatation catheter for coronary use, having a durable lubricious coating. The present invention satisfies that need.

## SUMMARY OF THE INVENTION

The present invention is directed to an improved vascular catheter having a lubricious coating thereon which retains its lubricious properties even after passage through tortuous vascular anatomy.

The vascular catheter of the present invention has a durable lubricious coating bonded thereon. Preferably, the coating comprises two layers, a first or base layer which is bonded to the catheter surface and a second or top layer bonded to the first layer which has the lubricious properties. The base layer is a polymer which forms a three-dimensional network of polymer units when cured. It is preferably partially cured to form an initial bond with the underlying catheter surface which is strong enough for subsequent processing. The second or lubricious material is then applied to the upper surface of the base layer to be thereby incorporated into the three-dimensional network. Final curing locks the second layer of lubricious material into the three-dimensional network in the upper portion of the base layer to thereby provide a lubricious coating having substantially improved durability. The lubricious coating has a coefficient of friction of at least 0.02 less than the coefficient of the catheter surface to which it is bonded.

The polymer material of the base layer is preferably a reactive polysiloxane, and a particularly suitable polysiloxane is a copolymer sold by Dow Corning as MDX-4-4119 which is described in U. S. Patent 3,574,673. The lubricious material of the top layer is preferably a polysiloxane oil which can easily be incorporated into the three-dimensional network of the upper surface of the base layer. A particularly suitable polysiloxane oil is 360 Medical Grade Silicone Oil sold by Dow Corning.

The first and second layers are preferably applied as solutions of the respective polysiloxane resin or oil in a non-aqueous solution containing at least 15% (by volume) of organic polar solvents. A mixture of methylene chloride and isopropyl alcohol is preferred but other organic polar solvents may also be used, including carbon tetrachloride, chlorbenzene, 1,1,2, trichloroethane, chloroform, ethyl acetate, and methy ethyl ketone. The preferred composition of the solutions are shown as follows:

| SOLUTION ID | % SOLUTE* | % METHYLENE CHLORIDE* | %ISOPROPYL ALCOHOL* |
|---|---|---|---|
| Base Layer | 2 - 10% | 10 - 40% | 50 - 90 |
| Top Layer | 5 - 15% | 10 - 40% | 50 - 85 |

\* All percentages are % by volume.

Preferably, the solutions for both the first and second coatings are wiped on with sponges or sponge-like applicators at room temperature and then subjected to infrared radiation. The IR radiation raises the temperature of the applied solution so as to evaporate the solvent and cures the polymerizable resin which remains on the catheter surface. The base layer is only partially cured so that the lubricious material of the second layer will be readily incorporated into the three-dimensional network. Irradiation after application of the second layer completes the polymerization of the resin, thereby locking in the lubricious material.

The catheters of the invention have a substantially lower coefficient of surface friction than any other catheters heretofore made and, in the case of balloon dilatation catheters, wings allow the deflated balloon to be folded about an inner member, as shown in FIG. 6B in U.S. Patent 4,323,071, much tighter than prior balloons to provide a lower effective profile. Due to the lubricious coating formed thereon, the dilatation catheters of the present invention pass lesions through which prior catheters of the same size could not pass and they can be advanced much farther into tortuous coronary vasculature than prior catheters of the same size. Moreover, dilatation balloons of the invention have little or no tendency to form dissections in the artery walls as the balloon is inflated to the high pressures encountered for dilatation procedures (e.g., up to 8 atmospheres or more). These and other advantages of the invention will become more apparent from the following detailed description thereof when taken in conjunction with the exemplary drawings.

## BRIEF DESCRIPTION OF THE DRAWINGS

FIGURE 1 illustrates a steerable low-profile dilatation catheter which has a coating thereon embodying features of the present invention;

FIG. 2 is an expanded partial cross section taken through the balloon shown in FIG. 1; and

FIG. 3 is a graphical representation of the reduction in profile afforded by the present invention.

## DETAILED DESCRIPTION OF THE INVENTION

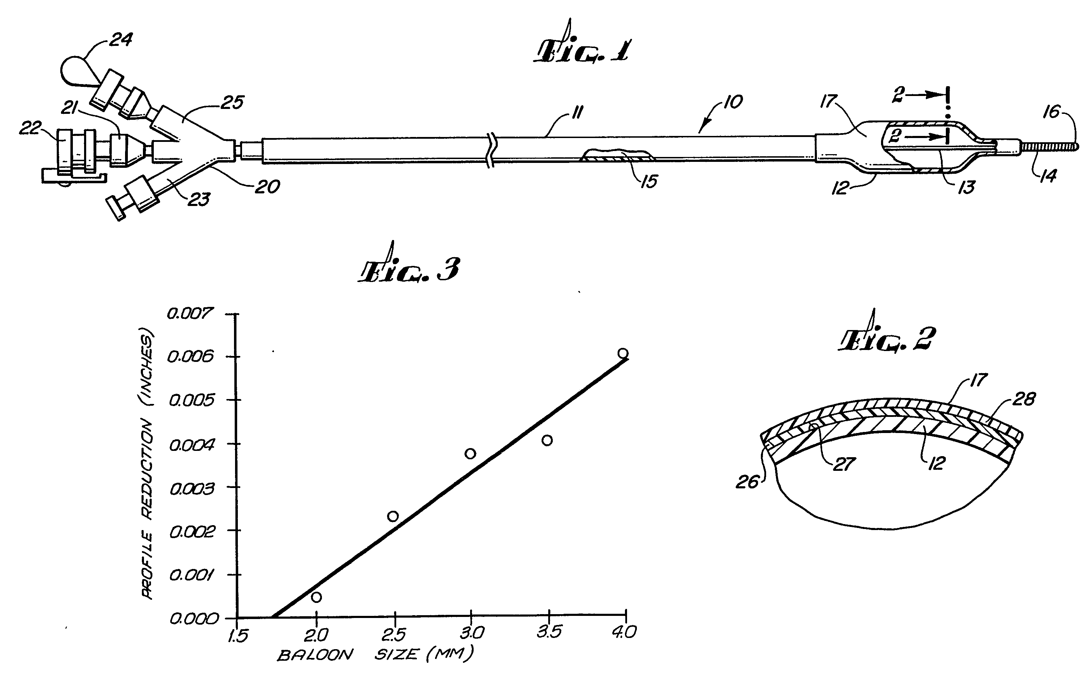

FIG. 1 illustrates a steerable fixed wire dilatation catheter 10 embodying features of the invention. The catheter 10 generally comprises a tubular member 11, an inflatable balloon 12 and a guiding member 13 extending through the balloon 12 and having a coiled spring 14 on the distal end thereof.

A three-arm adapter 20 is provided on the proximal end of the catheter 10. The guiding member 13 passes through arm 21 and is provided with a torquing knob 22 on the proximal end to steer the distal end of the catheter. Arm 23 is provided to supply inflation fluid to the balloon 12. A vent tube 24 extends through arm 25.

The tubular member 11 has an inner lumen 15 extending therethrough which is in fluid communication with the interior of balloon 12. A radiopaque rounded plug 16 is provided on the distal tip of the coil 14. A lubricious coating 17 is bonded onto the surface of the distal portion of catheter 10 in accordance with the invention to reduce the coefficient of friction thereof as well as to reduce the effective profile of the dilatation catheter, particularly the balloon 12.

FIG. 2, which is a cross-sectional view of the balloon wall, generally illustrates coating 17 in accordance with the invention. As indicated, the coating 17 comprises two layers, a first or base layer 26, preferably a reactive polysiloxane which is bonded to the surface 27 of the catheter 10, and a second or top layer 28, preferably a polysiloxane which has lubricious properties and which is bonded to the first or base layer 26.

The method of applying the first and second layers 26 and 28 generally comprises dissolving the coating materials in suitable non-aqueous solvents having substantial quantities of organic polar solvent, wiping the solution onto the surface of the catheter by means of a sponge or sponge-like material and then subjecting the coating to radiation, such as an infrared (IR) lamp, which drives off the solvent and polymerizes or otherwise bonds the solids dissolved in the solvents to the underlying substrate, which in the case of the first layer is the catheter and/or the balloon surface 27 and in the second instance would be the polymerized first layer. Preferably, the first layer is only partially cured and the second layer is subsequently applied to the upper surface of the partially cured layer to be incorporated into the three-dimensional network of polymer units. A final radiation treatment drives off the polar solvent of the second solution and completes the curing of the underlying first layer, thereby locking the lubricious solids in the second solution to the first layer to provide the long-lasting lubricious characteristics to the catheter.

The reactive polysiloxane material which forms the first layer preferably is a copolymer in which about 3 to 50 percent (by weight) of the dimethyl siloxane polymer units have reactive amino groups. The remainder is a polymerizable dimethylsiloxane which reacts therewith. Suitable copolymers are described in U. S. Patent 3,574,673. In addition to the amino alkyl siloxane described in U. S. Patent 3,574,673, other suitable amino polysiloxanes include N-(2-aminoethyl)3-amino propyl methyl dimethoxy silane, N-(2-aminoethyl)3-amino propyl dimethoxy silane, and 3-amino propyl methyldimethoxysilane. Suitable replacements for the preferred medical grade silicone oils include low molecular weight linear silanes, tetraalkoxy silanes, and organotris (trimethylsiloxy silanes).

## EXAMPLE

A plurality of balloon dilatation angioplasty catheters as shown in FIG. 1 formed from polyethylene in a conventional fashion having inflated balloon sizes of 2.0, 2.5, 3.0, 3.5, and 4.0 mm were coated in accordance with the present invention. The solution for forming the first layer contained 5 percent (by volume) of polysiloxane copolymer MDX-4-4119 (sold by Dow Corning), 19 percent (by volume) methylene chloride and 76% (by volume) isopropyl alcohol. It was applied to the catheter surfaces including the balloon surface by means of a sponge saturated with the solution and then the catheters were treated with IR radiation to drive off solvent and to cure solid resinous material therefrom. The solution for forming the second layer contained 10 percent (by volume) of 360 Medical Grade Fluid Silicone (sold by Dow Corning), 18 percent (by volume) methylene chloride, and 72 percent (by volume) isopropyl alcohol and was applied to the partially cured coating by means of a sponge which had been saturated with the solution. The catheters were then subjected to further IR radiation to complete the curing of the polymerizable resin in the first layer. Each of the catheters so coated was passed through a series of sizing holes in a profile block to determine the smallest size of the holes through which the catheters would pass. In each case, each of the above catheters so tested could be advanced through the holes in the profile block having diameters much smaller than the diameters of holes through which catheters of the same size without the coating could be pushed. The differences in profiles between coated and uncoated catheters of the same size is graphically illustrated in FIG. 3. As is evident, substantial reductions in profile are obtained even with smaller-sized catheters.

The catheters coated in accordance with the invention have been tested in human clinical evaluations and have been found to provide substantial improvements in the ability to advance such catheters through the coronary vasculature of a patient and to more readily cross stenoses than dilatation catheters not having such coatings thereon. Moreover, there was evidence of substantial reductions in the extent and severity of dissections when the dilatation balloons were inflated to elevated pressures during angioplasty procedures. A typical coefficient of friction with these coatings was about .041 which is to be compared with conventional catheter coefficients of about .069. The coatings formed are durable and do not lose their effective lubricity even when passing through the tortuous passageways of the coronary arteries or tight stenoses.

The coatings applied in accordance with the present invention generally range in thickness from about 0.03 to about 0.08 microns thick. The individual layers of the coating are generally about the same thickness, although a wide variation is permissible. The preferred curing media for the polysiloxane polymers is IR radiation, although the layers can be reacted with heat and other types of radiation. However, such other curing media may affect the time for curing as well as the soundness of the cured layer.

Various modifications can be made to the present invention. For example, while the invention has been described herein as a coating applied to the exterior of catheters, it can be effectively applied to the interior surfaces of catheters as well. Other modifications and improvements can be made to the invention without departing from the scope thereof.

## Claims

1. A dilatation catheter for angioplasty procedures which has an elongated catheter body and an inflatable, relatively inelastic balloon on the distal portion thereof, said balloon having a plurality of wings in a deflated condition folded around an inner member and having bonded onto the outer surface thereof a durable lubricious coating formed by a cross-linked polysiloxane polymer having incorporated therein a lubricious material to reduce the effective profile of the balloon and to minimize damage to the arterial lining during angioplasty procedures when the balloon is inflated.

2. The dilatation catheter of claim 1 wherein the lubricious coating comprises a base layer of cross-linked polymer bonded to the exterior surface of the balloon and lubricious material incorporated into the upper surface of said base layer.

3. The dilatation catheter of claim 2 wherein the lubricious material is a polysiloxane oil.

4. The dilatation catheter of claim 2 wherein the base layer has a three-dimensional network of polymer units in the upper portion thereof.

5. The dilatation catheter of claim 4 wherein the lubricious material is partially incorporated and locked into the three-dimensional network of polymer units in the upper portion of the base layer.

6. The dilatation catheter of any one of claims 1-5 wherein the polysiloxane polymer is a copolymer having from about 5 to about 50% of the polymeric units having reactive amino sites.

7. The dilatation catheter of any one of claims 1-6 wherein said durable lubricious coating is applied to essentially the entire distal portion of the catheter which is adapted to extend out the distal end of a guiding catheter.

8. The dilatation catheter of any one of claims 1-7 wherein the coating thickness ranges from about 0.03 to about 0.08 microns.

9. A dilatation catheter of any one of the preceding claims wherein the lubricious coating bonded onto the surface has a coefficient of friction at least 0.02 less than the coefficient of friction of the balloon surface.

10. The dilatation catheter of claim 9 wherein the lubricious coating comprises a base layer of cross-linked polysiloxane polymer bonded to the exterior surface of the balloon and a top lubricious layer bonded to the upper surface of said base layer.

11. The dilatation catheter of claim 10 wherein the polysiloxane polymer is a copolymer having from about 5 to about 50% of the polymeric units having reactive amino sites.

12. The dilatation catheter of claim 10 wherein the lubricious material is a polysiloxane oil.

13. The dilatation catheter of claim 10 wherein the base layer has a three-dimensional network of polymer units in the upper portion thereof.

14. The dilatation catheter of claim 10 wherein the lubricious layer is partially incorporated and locked into the three-dimensional network of polymer units in the upper portion of the base layer.

15. The dilatation catheter of claim 9 wherein the coating thickness on the balloon ranges from about 0.03 to about 0.08 microns.

16. A method of forming a durable lubricious coating on the surface of a vascular catheter comprising:

a) applying a first layer of curable resin onto the surface of the vascular catheter;

b) applying curing medium to the applied resin to at least partially cure the resin and bond the resin to the catheter surface;

c) applying a second layer of a lubricious material onto the first layer of resin, incorporating lubricious material into at least the top surface of said first layer; and

d) applying further curing medium to the resin of the first layer to thereby bond lubricious material of the second layer to the first layer.

17. The method of claim 16 wherein the curable resin and the lubricious material are applied as solutions with one or more organic polar solvents.

18. The method of claim 17 wherein the polar solvents are selected from the group consisting of methylene chloride, isopropyl alcohol, and mixtures thereof.

19. The method of claim 18 wherein the polar solvent contains from about 10 to about 40 percent (by volume) methylene chloride and from about 60 to about 90 percent (by volume) isopropyl alcohol.

20. The method of any one of claims 16-19 wherein the curable resin is a polysiloxane copolymer having from about 5 to about 50 percent of the polymeric units having amino reaction sites.

21. The method of claim 16 wherein the lubricious oil is a dimethyoxy siloxane.

22. The method of claim 17 wherein the solution used to form the first layer contains at least 2 percent (by volume) of curable polysiloxane.

23. The method of claim 22 wherein the polymer is cured by infrared radiation.

24. The method of claim 16 wherein the first and second solutions are applied to the catheter surface by wiping such solutions onto the surface.

25. The method of claim 24 wherein the solutions are wiped onto the substrates by sponges or sponge-like bodies having said solutions absorbed therein.

Neu eingereicht / Newly fil
Nouvellement déposé

**Fig. 1**

**Fig. 2**

**Fig. 3**

BALOON SIZE (MM)

PROFILE REDUCTION (INCHES)

| | DOCUMENTS CONSIDERED TO BE RELEVANT | | EP 90101510.7 |
|---|---|---|---|

| Category | Citation of document with indication, where appropriate, of relevant passages | Relevant to claim | CLASSIFICATION OF THE APPLICATION (Int Cl⁵) |
|---|---|---|---|
| P,Y | EP - A2 - 0 329 041 (BECTON DICKINSON) <br> * Totality; especially page 3, lines 25-41; page 4, line 36 - page 5, line 4; page 5, line 52 - page 6, line 2; claims 1-6,9 * | 1-5 | A 61 M 29/02 <br> A 61 M 25/10 <br> A 61 L 29/00 |
| A | -- | 8,9,16 | |
| Y | EP - A2 - 0 266 957 (C.R.BARD INC.) <br> * Fig 1,5; abstract; column 7, lines 9-17; column 9, lines 3,4 * | 1-5 | |
| A | US - A - 3 962 519 (W. RÜSCH et al.) <br> * Totality; especially fig. 1; column 2, line 55 - column 3, line 16; column 5, lines 16-30; column 7, lines 24-36; claims 1,3,5,6 * | 1,16 | |
| | -- | | **TECHNICAL FIELDS SEARCHED (Int Cl⁵)** |
| A | US - A - 4 666 437 (H.R.LAMBERT) <br> * Totality; especially abstract; claims 1,5,9 * | 16 | A 61 L 29/00 <br> A 61 M 25/00 <br> A 61 M 29/00 |
| A | US - A - 4 664 657 (V.A. WILLIAMITIS et al.) <br> * Totality; especially abstract; claims 1,6 * | 1,16 | |
| D,A | US - A - 4 771 778 (C.E. MAR) <br> * Fig. 1,3; abstract * | 1 | |
| P,X | US - A - 4 838 876 (E.W. WONG et al.) <br> * Totality; abstract; column | 1-5 | |

The present search report has been drawn up for all claims

| Place of search | Date of completion of the search | Examiner |
|---|---|---|
| VIENNA | 15-05-1990 | LUDWIG |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | 1, lines 26-32; column 2, line 49 − column 3, line 20; column 4, lines 1-30; claims 1-12,15 *<br><br>---- | 8,9,16 | |

RECHERCHIERTE SACHGEBIETE (Int Cl⁵)

Der vorliegende Recherchenbericht wurde für alle Patentansprüche erstellt.

| Recherchenort | Abschlußdatum der Recherche | Prüfer |
|---|---|---|
| VIENNA | 15-05-1990 | LUDWIG |